# EUROPEAN PATENT SPECIFICATION

(11) **EP 2 997 879 B1**
(45) Date of publication and mention of the grant of the patent: **13.03.2019**
(21) Application number: 14775581.3
(22) Date of filing: 10.01.2014
(51) Int. Cl.: A61B 1/04, A61B 1/00, A61B 90/00, A61B 1/005, A61B 34/30, A61B 34/37

(54) **MASTER-SLAVE SYSTEM**
MASTER-SLAVE-SYSTEM
SYSTÈME MAÎTRE-ESCLAVE

(30) Priority: 29.03.2013 US 201361806500 P
(43) Date of publication of application: 23.03.2016
(73) Proprietor: Olympus Corporation, Tokyo 192-8507 (JP)
(72) Inventor: OGAWA, Ryohei, Hachioji-shi, Tokyo 192-8507 (JP); KISHI, Kosuke, Hachioji-shi, Tokyo 192-8507 (JP)
(74) Representative: Gunzelmann, Rainer
(86) International application number: PCT/JP2014/050264
(87) International publication number: WO 2014/156217

(56) References cited:
- WO-A1-2010/055745
- JP-A- 2007 175 502
- JP-A- 2007 252 921
- JP-A- 2008 155 030
- JP-A- 2008 228 967
- US-A1- 2008 154 091
- US-A1- 2008 234 866
- US-A1- 2010 318 100

## Description

### {Technical Field}

The present invention relates to a master-slave system.

### {Background Art}

In the related art, there is a known master input device in master-slave systems that has a similar shape to a slave device (for example, see PTL 1).

In a master-slave system of PTL 1, the slave device is an endoscope that has an observation optical system and two treatment tools on a distal-end surface of an elongated insertion portion, and the master device has a plurality of joints that allow the corresponding bending portions of the insertion portion to perform bending movements, a handle that causes, through a rotating movement thereof, the insertion portion to perform a twisting movement, and two grip portions that are disposed at the ends of the handle, that are manipulated by both hands so as to move the two treatment tools, and that have similar shapes to the treatment tools.

Further prior art related to a master-slave system includes the following documents: US Patent Application Publication No. 2010/318100 A1, US Patent Application Publication No. 2008/154091 A1, US Patent Application Publication No. 2008/234866 A1.

### {Citation List}

### {Patent Literature}

{PTL 1} Publication of Japanese Patent No. 4608601

### {Summary of Invention}

### {Technical Problem}

However, in the master-slave system of PTL 1, there is a disadvantage in that, when the handle of the master device is manipulated to cause the distal end of the insertion portion to perform a rotating movement, because the observation optical system, which is provided on the distal-end surface of the insertion portion on which the treatment tools are provided, is also rotated at the same time, the position of the handle of the master device and the positions of the treatment tools on the monitor are mismatched. Specifically, even when the distal-end surface is rotated by rotating the handle, the relative positional relationship between the observation optical system and the treatment tools, which are provided on the distal-end surface, is not changed; therefore, in an image acquired by the observation optical system and displayed on the monitor, the positions of the treatment tools are never changed, and thus the position of the handle and the positions of the treatment tools on the monitor are mismatched.

The present invention has been made in view of the above-described circumstances, and an object thereof is to provide a master-slave system capable of maintaining the association between the position of a handle and the position of a treatment tool on a monitor through manipulation of the handle of a master device.

### {Solution to Problem}

In order to achieve the above-described object, the present invention provides the following solutions.

According to one aspect, the present invention provides a master-slave system including the features of claim 1. A master-slave system may include: a slave device that includes an observation optical system that photographs a subject and a treatment portion that protrudes from a surface on which the observation optical system is provided and that is at least partially photographed by the observation optical system together with the subject; a master device that includes a handle that is supported so as to be capable of rotating about a predetermined axis and a treatment manipulation portion that is provided on the handle; a control portion that associates rotation of the handle of the master device with rotation of the slave device about an axis intersecting the surface and that associates manipulation of the treatment manipulation portion with movement of the treatment portion; an image processing portion that generates a corrected image obtained by rotating an image acquired by the observation optical system by a rotation angle of the slave device about the axis intersecting the surface; and a monitor that displays the corrected image generated by the image processing portion.

According to this aspect, when the handle of the master device is rotated about the predetermined axis, the control portion rotates the slave device about the axis intersecting the surface on which the observation optical system is provided, by the angle corresponding to the rotation angle of the handle. Accordingly, the direction in which the observation optical system observes the subject is changed. Because the treatment portion protrudes from the surface on which the observation optical system is provided, the treatment portion is simultaneously rotated in the same direction when the observation optical system is rotated.

Furthermore, when the treatment manipulation portion, which is provided on the handle of the master device, is manipulated, the control portion moves the treatment portion by the amount corresponding to the manipulation of the treatment manipulation portion. Accordingly, it is possible to treat the subject with the treatment portion from the direction in which the observation optical system performs observation.

In this case, when the handle is rotated, the image processing portion generates a corrected image, and the generated corrected image is displayed on the monitor. Because the corrected image is obtained by rotating an image acquired by the observation optical system by the rotation angle of the slave device that has been rotated by rotating the handle, it is possible to perform display on the monitor such that the subject is not rotated, and the treatment portion is rotated with respect to the subject.

Specifically, because the treatment portion on the monitor is rotated in the same direction as the rotation direction of the handle of the master device, the operator, who performs treatment while viewing the monitor, can perform intuitive manipulation. Furthermore, it is possible to match the position of the handle with the position of the treatment portion on the monitor, thus easily recognizing the association between the handle and the treatment portion even when manipulation is performed again some time after the last manipulation.

Furthermore, according to another aspect, the present invention provides a master-slave system including the features of claim 2. A master-slave system may include: a slave device that includes an observation optical system that photographs a subject and a treatment portion that protrudes from a surface on which the observation optical system is provided and that is at least partially photographed by the observation optical system together with the subject; a master device that includes a handle that is supported so as to be capable of rotating about a predetermined axis and a treatment manipulation portion that is provided on the handle; a control portion that associates rotation of the handle of the master device with rotation of the slave device about an axis intersecting the surface and that associates manipulation of the treatment manipulation portion with movement of the treatment portion; an image processing portion that generates a corrected image obtained by rotating an image acquired by the observation optical system by a rotation angle of the handle; and a monitor that displays the corrected image generated by the image processing portion.

Moreover, according to another aspect, the present invention provides a master-slave system including the features of claim 3.

According to still another aspect, the present invention provides a master-slave system including the features of claim 4.

According to the aspects above, when the handle is rotated, the image processing portion generates a corrected image, and the generated corrected image is displayed on the monitor. Because the corrected image is obtained by rotating an image acquired by the observation optical system by the rotation angle of the handle, the treatment portion on the monitor can be rotated in the same direction as the rotation direction of the handle of the master device, and the operator, who performs treatment while viewing the monitor, can perform intuitive manipulation. Furthermore, it is possible to match the position of the handle with the position of the treatment portion on the monitor, thus easily recognizing the association between the handle and the treatment portion even when manipulation is performed again some time after the last manipulation.

In the above-described aspects, the image processing portion may generate the corrected image obtained by rotating an image acquired by the observation optical system about the center of the image.

By doing so, the corrected image can be generated through simple processing.

Furthermore, in the above-described aspects, the image processing portion may generate the corrected image obtained by rotating an image acquired by the observation optical system about an axis that is fixed relative to the subject in the image.

By doing so, it is possible to display, on the monitor, a corrected image in which the subject is moved as little as possible, whereas the treatment portion is moved, and to generate an easy-to-view corrected image.

Furthermore, in the above-described aspects, when the handle is rotated up to a set angle, the control portion may switch from the corrected image on the monitor to a re-corrected image obtained by additionally rotating the corrected image by a predetermined correction angle from which the absolute value of the rotation angle of the handle can be subtracted, release the association between rotation of the handle of the master device and rotation of the slave device about the axis intersecting the surface, and resume the association when the handle is moved by an angle whose difference from the correction angle is equal to or lower than a predetermined threshold.

By doing so, because the posture of the operator becomes cramped when the rotation angle of the handle is increased, the image displayed on the monitor is changed from a corrected image to a re-corrected image when the handle is rotated up to the set angle, thereby making it possible to return the treatment portion on the monitor to a position whose rotation angle is smaller than that of the actual position of the handle. In this state, the association between the master device and the slave device is released to produce a situation in which the slave device is not moved even when the master device is manipulated, thereby maintaining the slave device at the position where it has been rotated up to the set angle.

Then, in this state, the handle is moved by an angle whose difference from the correction angle is equal to or lower than the predetermined threshold, thereby making it possible to approximately match the position of the handle of the master device with the position of the treatment portion on the monitor. After that, the association therebetween is resumed, thereby allowing the handle to be manipulated in the positional relationship in which it is associated with the treatment portion displayed on the monitor, within an easy-to-manipulate angular range up to the set angle, and also allowing the slave device to be rotated over a wide angular range beyond the set angle.

Furthermore, in the above-described aspects, the control portion may release the association between rotation of the handle and rotation of the slave device about the axis intersecting the surface, with the handle having been rotated, rotate, when the handle is then moved by a arbitrary movement angle, the corrected image displayed on the monitor by the movement angle, and resume the association.

By doing so, the posture of the operator becomes cramped when the rotation angle of the handle is increased; therefore, with the handle having been rotated up to a arbitrary angle, the association between the master device and the slave device is released to produce a situation in which the slave device is not moved even when the master device is manipulated, thereby maintaining the slave device at the rotated position.

In this state, with the handle having been moved by the arbitrary movement angle, the association is resumed, thereby associating the state in which the slave device has been rotated with the state in which the handle has been moved from the set angle by the movement angle. Also, the corrected image is rotated by the movement angle by the image processing portion, thereby matching the position of the treatment portion displayed on the monitor with the position of the handle of the master device.

Accordingly, it is possible to allow the handle to be manipulated in the positional relationship in which it is associated with the treatment portion displayed on the monitor, within an easy-to-manipulate angular range, and also to allow the slave device to be rotated over a wide angular range.

Furthermore, according to a comparative example, a master-slave system may include: a slave device that includes an observation optical system that photographs a subject and a treatment portion that protrudes from a surface on which the observation optical system is provided and that is at least partially photographed by the observation optical system together with the subject; a master device that includes a handle that is supported so as to be capable of rotating about a predetermined axis and a treatment manipulation portion that is provided on the handle; a control portion that associates rotation of the handle of the master device with rotation of the slave device about an axis intersecting the surface and that associates manipulation of the treatment manipulation portion with movement of the treatment portion; a monitor that displays an image acquired by the observation optical system; and an input portion that disables manipulation of the treatment manipulation portion, in which the control portion releases the association between rotation of the handle and rotation of the slave device about the axis intersecting the surface, with the manipulation of the treatment manipulation portion having been disabled by the input portion, resumes the association when the handle is located at a position corresponding to the treatment portion displayed on the monitor, and enables the manipulation of the treatment manipulation portion.

According to this comparative example, when the handle of the master device is rotated about the predetermined axis, the control portion rotates the slave device about the axis intersecting the surface on which the observation optical system is provided, by the angle corresponding to the rotation angle of the handle. Accordingly, the direction in which the observation optical system observes the subject is changed. Because the treatment portion protrudes from the surface on which the observation optical system is provided, the treatment portion is simultaneously rotated in the same direction when the observation optical system is rotated.

The treatment portion is rotated when the observation optical system is rotated; therefore, in an image displayed on the monitor, the treatment portion is always maintained at the same position, and only the subject is moved.

Furthermore, when the treatment manipulation portion, which is provided on the handle of the master device, is manipulated, the control portion moves the treatment portion by the amount corresponding to the manipulation of the treatment manipulation portion. Accordingly, it is possible to treat the subject with the treatment portion from the direction in which the observation optical system performs observation.

In this case, with the handle having been rotated up to a arbitrary rotation angle (movement angle), when the operator disables the manipulation of the treatment manipulation portion through the input portion, the treatment portion is locked so as not to be moved even when the treatment manipulation portion is manipulated. In this state, the control portion releases the association between rotation of the handle and rotation of the slave device, thereby producing a situation in which the slave device is not moved even when the handle is manipulated, and maintaining the slave device at the position where it has been rotated by the movement angle.

Then, when the operator manipulates the handle to locate the handle at the position corresponding to the treatment portion on the monitor, the control portion resumes the association; therefore, the handle and the slave device are associated while being mismatched by the movement angle, whereas the position of the handle and the position of the treatment portion on the monitor are matched. Accordingly, it is possible to allow the handle to be manipulated in the positional relationship in which it is associated with the treatment portion displayed on the monitor, within an easy-to-manipulate angular range, and also to allow the slave device to be rotated over a wide angular range beyond the movement angle.

Furthermore, according to still another aspect, the present invention provides a master-slave system including the features of claim 7. A master-slave system may include: a slave device that includes an observation optical system that photographs a subject and a treatment portion that protrudes from a surface on which the observation optical system is provided and that is at least partially photographed by the observation optical system together with the subject; a master device that includes a handle that is supported so as to be capable of rotating about a predetermined axis and a treatment manipulation portion that is provided on the handle; a control portion that associates rotation of the handle of the master device with rotation of the slave device about an axis intersecting the surface and that associates manipulation of the treatment manipulation portion with movement of the treatment portion; and a monitor that is fixed to the handle and that displays an image acquired by the observation optical system.

According to this aspect, when the handle of the master device is rotated about the predetermined axis, the control portion rotates the slave device about the axis intersecting the surface on which the observation optical system is provided, by the angle corresponding to the rotation angle of the handle. Accordingly, the direction in which the observation optical system observes the subject is changed. Because the treatment portion protrudes from the surface on which the observation optical system is provided, the treatment portion is simultaneously rotated in the same direction when the observation optical system is rotated.

The treatment portion is rotated when the observation optical system is rotated; therefore, in an image displayed on the monitor, the treatment portion is always maintained at the same position, and only the subject is moved. In this case, because the monitor is fixed to the handle, the monitor is also rotated together with the handle. Accordingly, the treatment portion on the monitor is located at the same rotation-angle position as the rotation-angle position of the handle of the master device; therefore, the operator, who performs treatment while viewing the monitor, can perform intuitive manipulation.

In the above-described aspect, the control portion may release the association between rotation of the handle of the master device and rotation of the slave device about the axis intersecting the surface when the handle is rotated from a standard position up to a set angle and resume the association when the handle is returned to the standard position.

By doing so, because the posture of the operator becomes cramped when the rotation angle of the handle is increased, at a point in time at which the handle has been rotated up to the set angle, the association between the master device and the slave device is released to produce a situation in which the slave device is not moved even when the master device is manipulated, thereby maintaining the slave device at the position where it has been rotated up to the set angle.

In this case, when the handle is returned to the standard position, the control portion resumes the association, thus associating the state in which the slave device is rotated up to the set angle with the state in which the handle is located at the standard position. Because the position of the treatment portion displayed on the monitor is always matched with the position of the handle, it is possible to allow the handle to be manipulated in the positional relationship in which it is associated with the treatment portion displayed on the monitor, within an easy-to-manipulate angular range from the standard position to the set angle, and also to allow the slave device to be rotated over a wide angular range beyond the set angle.

As an example, a driving method for a master-slave system equipped with a slave device that includes an observation optical system that photographs a subject and a treatment portion that protrudes from a surface on which the observation optical system is provided and that is at least partially photographed by the observation optical system together with the subject; and a master device that includes a handle that is supported so as to be capable of rotating about a predetermined axis and a treatment manipulation portion that is provided on the handle may include: a movement step of rotating the slave device about an axis intersecting the surface according to rotation of the handle of the master device; an image generating step of generating a corrected image obtained by rotating an image acquired by the observation optical system by a rotation angle of the slave device about the axis intersecting the surface; and a display step of displaying the corrected image generated in the image generating step on a monitor.

As a further example, a driving method for a master-slave system equipped with a slave device that includes an observation optical system that photographs a subject and a treatment portion that protrudes from a surface on which the observation optical system is provided and that is at least partially photographed by the observation optical system together with the subject; and a master device that includes a handle that is supported so as to be capable of rotating about a predetermined axis and a treatment manipulation portion that is provided on the handle may include: a movement step of rotating the slave device about an axis intersecting the surface according to rotation of the handle of the master device; an image generating step of generating a corrected image obtained by rotating an image acquired by the observation optical system by a rotation angle of the handle; and a display step of displaying the corrected image generated in the image generating step on a monitor.

In the above-described examples, in the image generating step, the corrected image obtained by rotating an image acquired by the observation optical system about the center of the image may be generated.

Furthermore, in the above-described examples, in the image generating step, the corrected image obtained by rotating an image acquired by the observation optical system about an axis that is fixed relative to the subject in the image may be generated.

Furthermore, in the above-described examples, it is possible to further include: a release step of switching, when the handle is rotated up to a set angle, an image displayed on the monitor to a re-corrected image obtained by rotating the image by, in addition to the rotation angle of the handle, a predetermined correction angle from which the absolute value of the rotation angle of the handle can be subtracted and of releasing association between rotation of the handle of the master device and rotation of the slave device about the axis intersecting the surface; and a resumption step of resuming the association when the handle is moved by the correction angle after the release step and of resuming display of the corrected image on the monitor.

Furthermore, in the above-described examples, it is possible to further include: a release step of releasing association between rotation of the handle and rotation of the slave device about the axis intersecting the surface, with the handle having been rotated; and a resumption step of rotating, when the handle is moved by a arbitrary movement angle after the release step, the corrected image displayed on the monitor by the movement angle and of resuming the association.

### {Advantageous Effects of Invention}

According to the present invention, an advantageous effect is afforded in that the association between the position of the handle and the positions of the treatment tools on the monitor can be maintained through manipulation of the handle of the master device.

### {Brief Description of Drawings}

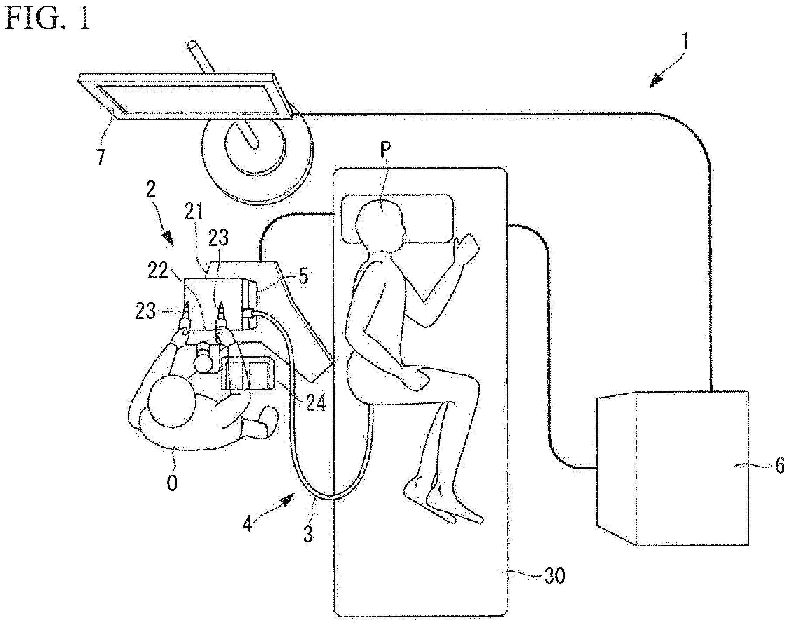

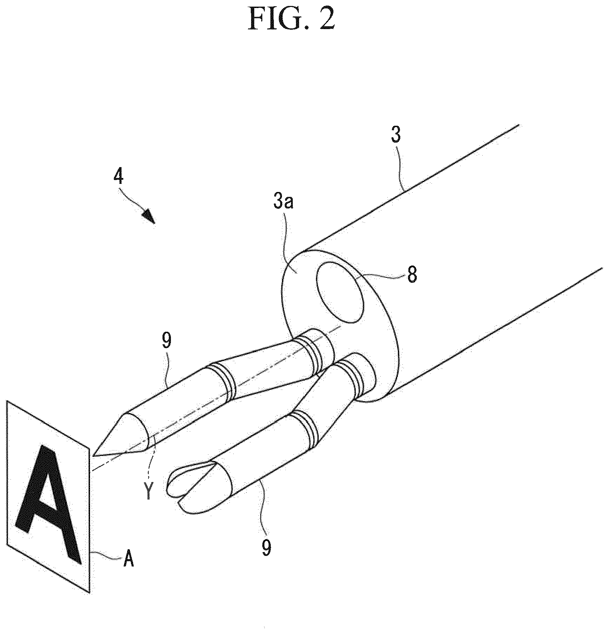

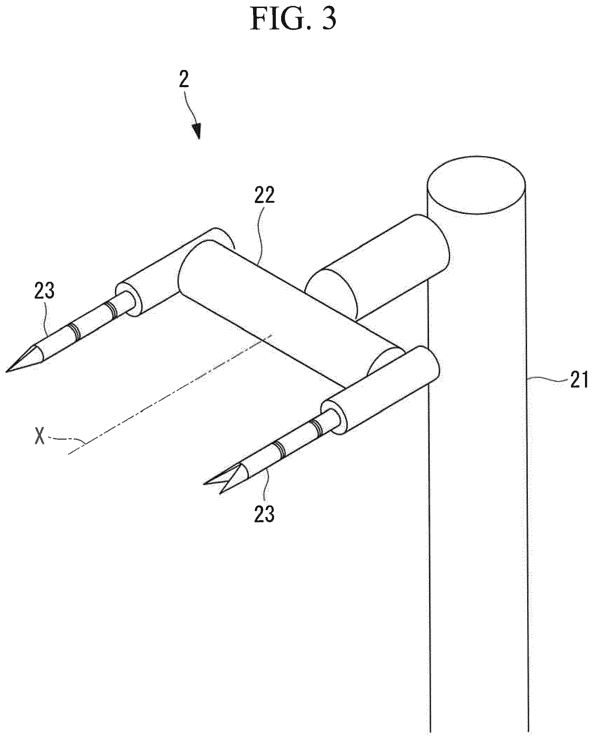

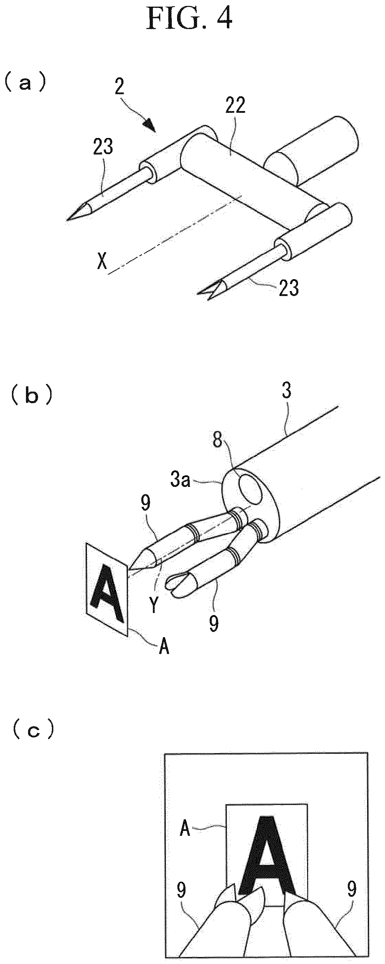

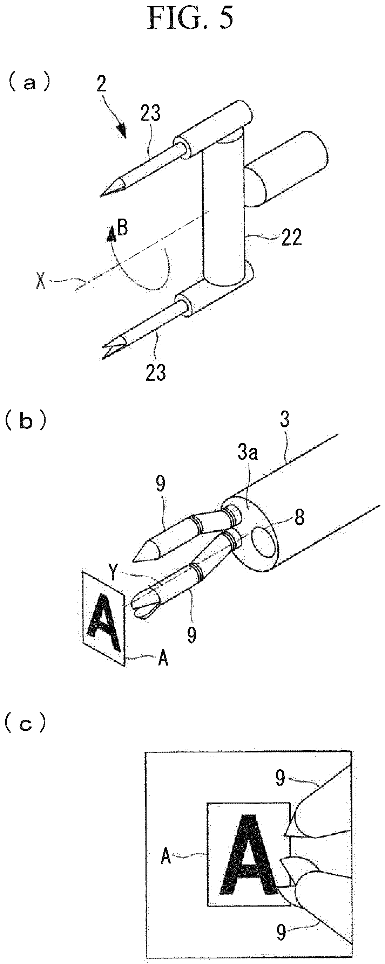

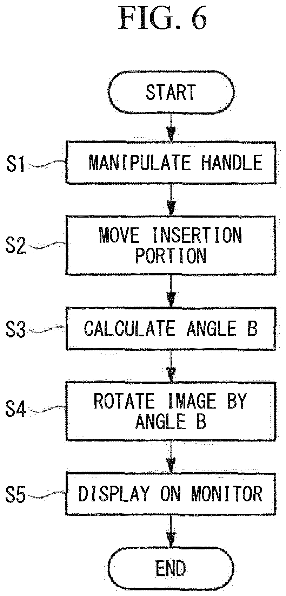

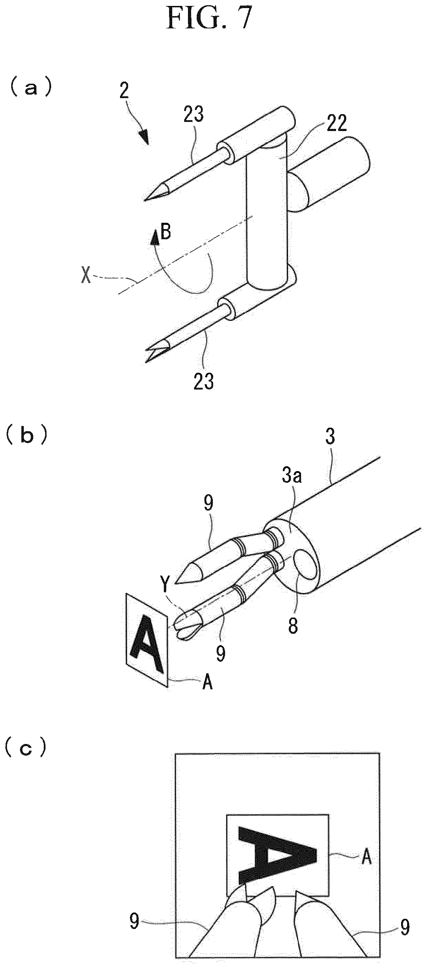

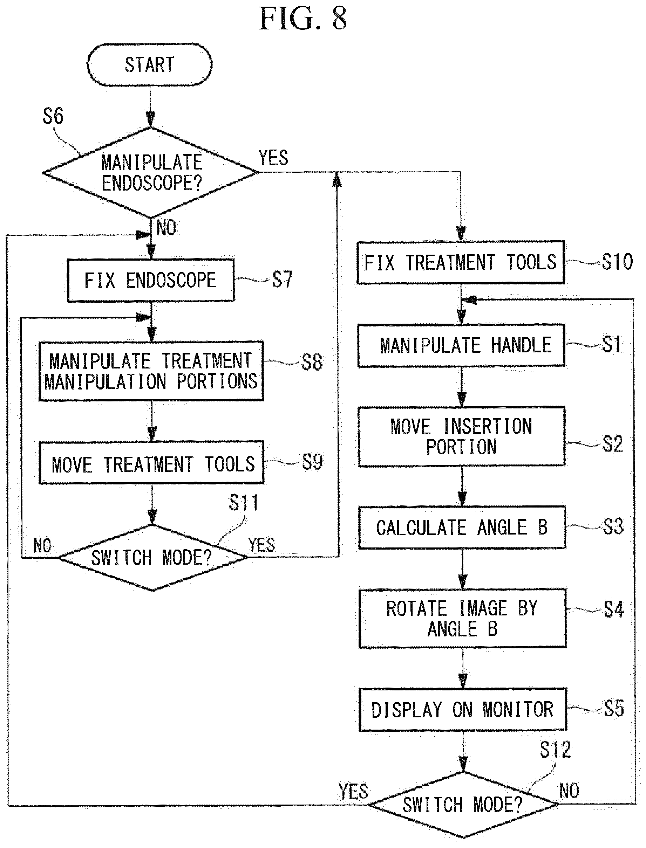

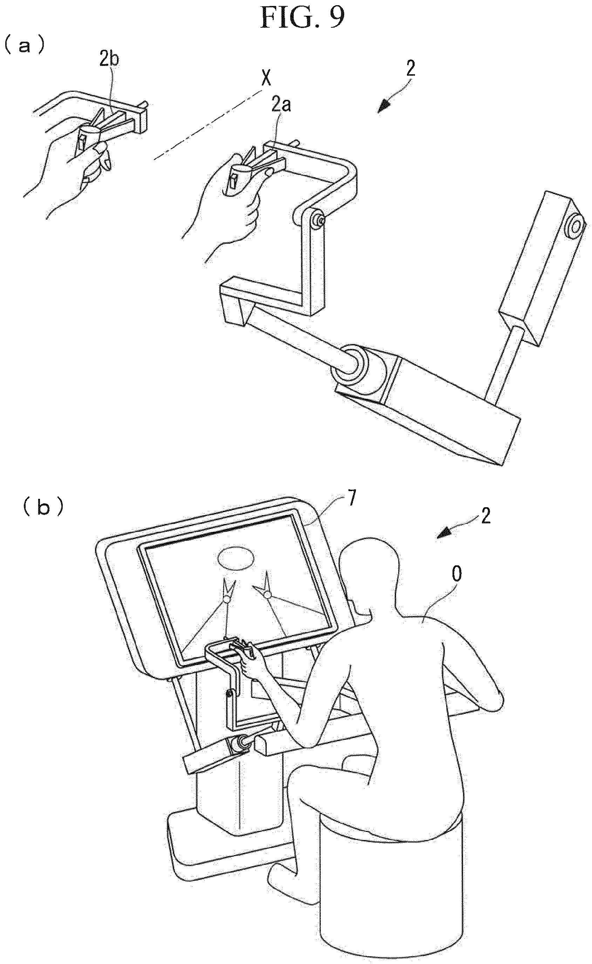

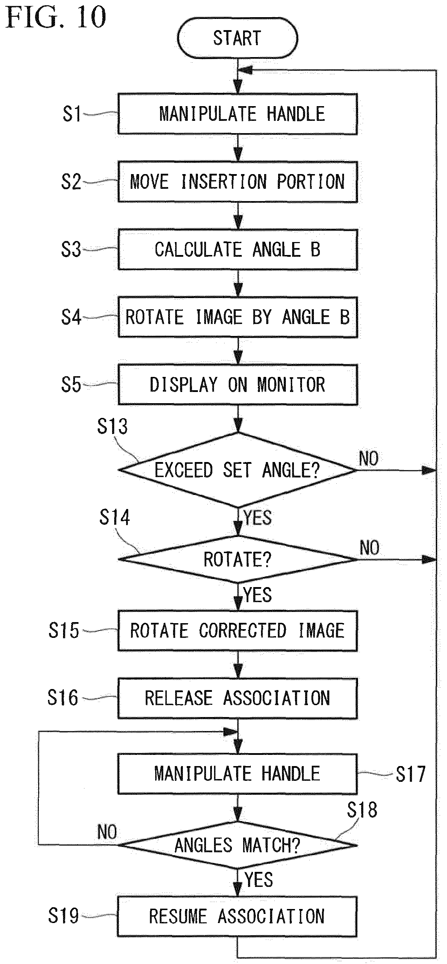

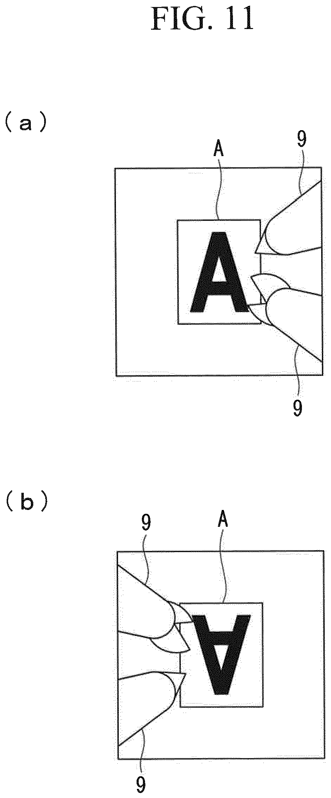

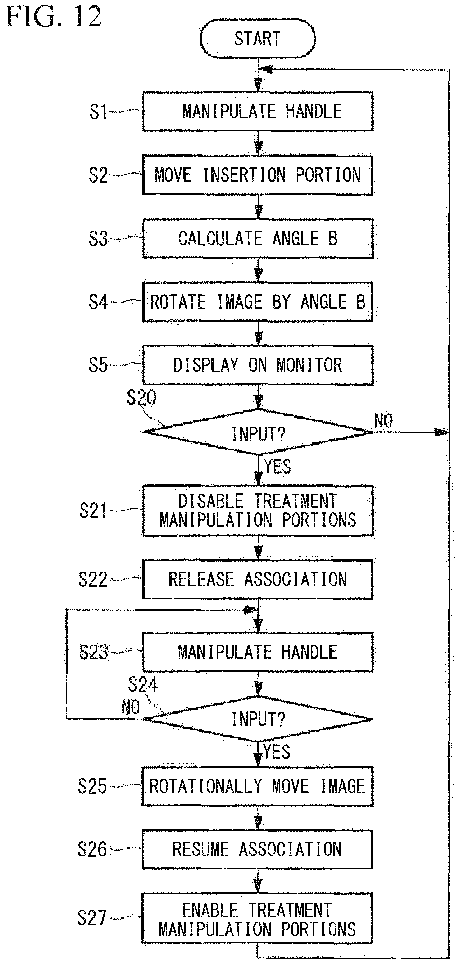

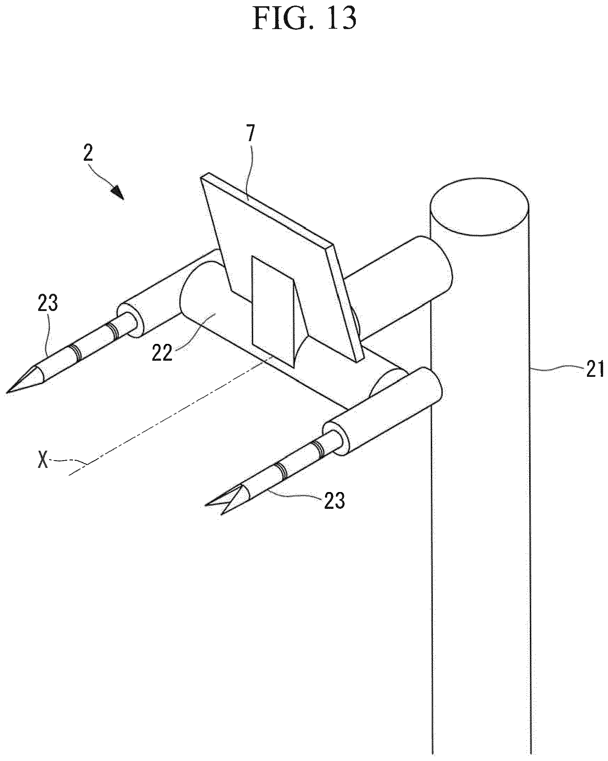

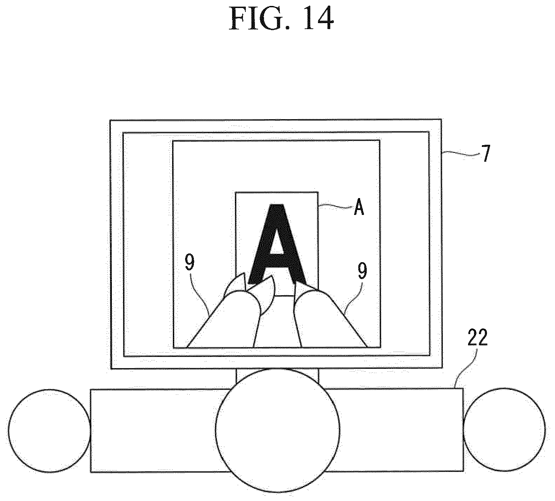

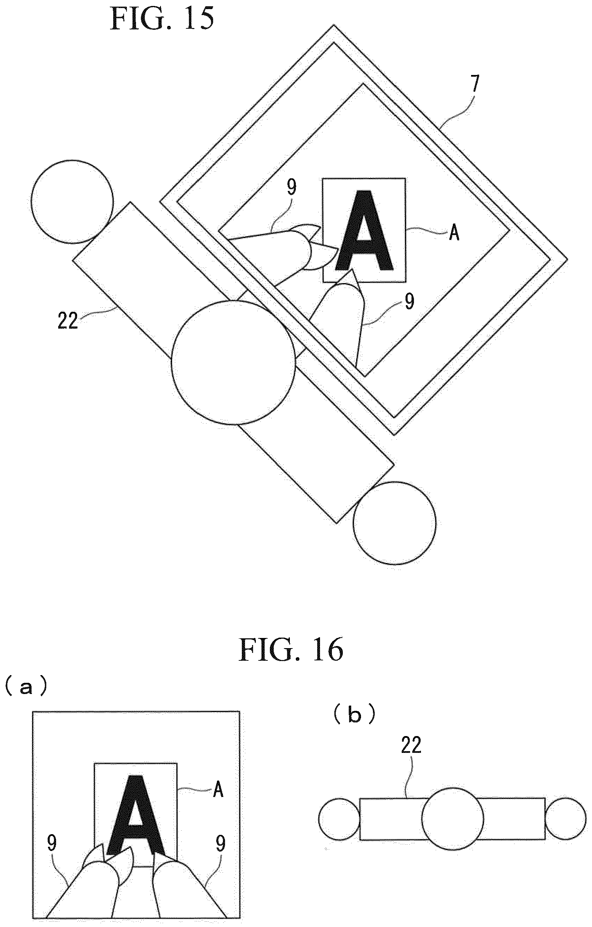

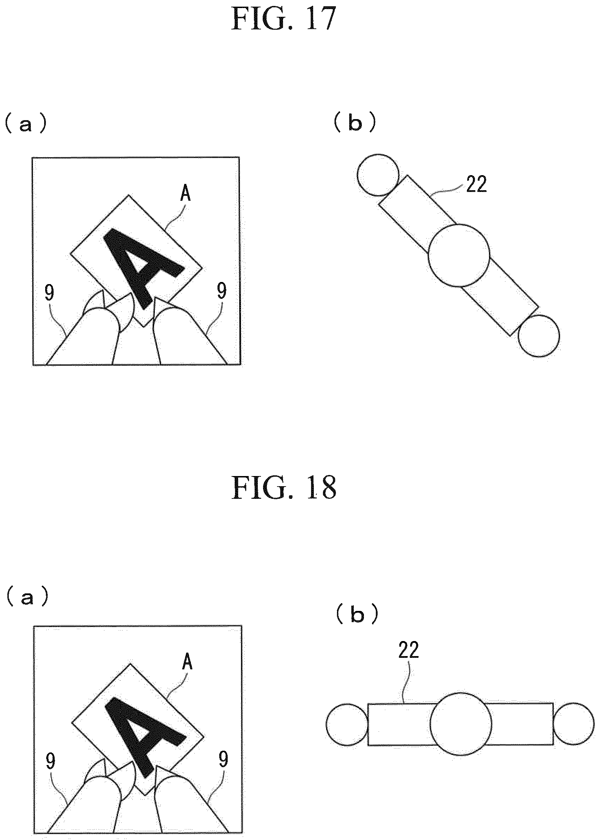

{Fig. 1} Fig. 1 is a view showing the overall configuration of a master-slave system according to a first embodiment of the present invention.
{Fig. 2} Fig. 2 is a perspective view showing a distal end of an insertion portion of an endoscope serving as a slave device of the master-slave system shown in Fig. 1.
{Fig. 3} Fig. 3 is a perspective view showing a master device of the master-slave system shown in Fig. 1.
{Fig. 4} Fig. 4 is a view showing (a) the master device, (b) the slave device, and (c) an image on a monitor, in a state in which the master-slave system shown in Fig. 1 is located at a standard position.
{Fig. 5} Fig. 5 is a view showing (a) the master device, (b) the slave device, and (c) the image on the monitor, in a state in which a handle of the master-slave system shown in Fig. 1 is rotated from the standard position shown in Fig. 4 up to a set angle.
{Fig. 6} Fig. 6 is a flowchart for explaining the movement of the master-slave system shown in Fig. 1.
{Fig. 7} Fig. 7 is a view showing (a) the master device, (b) the slave device, and (c) the image on the monitor, in a state in which the handle of a conventional master-slave system is rotated.
{Fig. 8} Fig. 8 is a flowchart for explaining a modification of the movement of the master-slave system shown in Fig. 1.
{Fig. 9} Fig. 9 is a perspective view showing (a) an example handle and (b) an example master device, for explaining a modification of the master device of the master-slave system shown in Fig. 1.
{Fig. 10} Fig. 10 is a flowchart for explaining the movement of a master-slave system according to a second embodiment of the present invention.
{Fig. 11} Fig. 11 is a view showing (a) an example image acquired when a handle is rotated up to a set angle and (b) an example image acquired when the handle is further rotated by 180°, for explaining the movement of the master-slave system shown in Fig. 10.
{Fig. 12} Fig. 12 is a flowchart for explaining the movement of a master-slave system according to a comparative example.
{Fig. 13} Fig. 13 is a perspective view showing a master device of a master-slave system according to a third embodiment of the present invention.
{Fig. 14} Fig. 14 is a view showing the handle and the image on the monitor, for showing a state in which the master-slave system shown in Fig. 13 is located at the standard position.
{Fig. 15} Fig. 15 is a view showing the handle and the image on the monitor, for showing a state in which the handle of the master-slave system shown in Fig. 13 is rotated from the standard position.
{Fig. 16} Fig. 16 is a view showing (a) an image on the monitor and (b) the position of the handle, in a state in which a master-slave system according to a modification of the present invention is located at the standard position.
{Fig. 17} Fig. 17 is a view showing (a) an image on the monitor and (b) the position of the handle, in a state in which the handle of the master-slave system shown in Fig. 16 is rotated from the standard position.
{Fig. 18} Fig. 18 is a view showing (a) an image on the monitor and (b) the position of the handle, in a state in which only the handle of the master-slave system shown in Fig. 16 is returned to the standard position.

The comparative example, as well as the method steps that are described below, only represent background that is useful for understanding the present invention, but do not form part of the same.

### {Description of Embodiments}

A master-slave system 1 according to a first embodiment of the present invention will be described below with reference to the drawings.

As shown in Fig. 1, the master-slave system 1 of this embodiment is an endoscope system and includes a master device 2 that is manipulated by an operator O, an endoscope 4 that serves as a slave device, a driving portion 5 that drives the endoscope 4, a control portion 6 that controls the driving portion 5, and a display portion (monitor) 7 that displays an image acquired by the endoscope 4.

The endoscope 4 has a flexible insertion portion 3 to be inserted into a body of a patient P, for example, into a soft organ, such as the large intestine.

As shown in Fig. 2, an objective lens 8 of an observation optical system is mounted on a distal-end surface 3a of the insertion portion 3, and two treatment tools (treatment portions) 9 that protrude forward from the distal-end surface 3a are also provided thereon. The treatment tools 9 each have an articulated structure. Furthermore, a plurality of bending portions (not shown) are provided in the vicinity of a distal end of the insertion portion 3. With a combination of bends of the bending portions, the distal-end surface 3a of the insertion portion 3 can be moved in any direction.

At a base end of the insertion portion 3, the driving portion 5 performs driving for an inserting movement of the insertion portion 3 of the endoscope 4, a bending movement of the insertion portion 3, and a twisting movement of the insertion portion 3.

As shown in Figs. 1 and 3, the master device 2 of this embodiment has: a handle 22 that is supported so as to be capable of rotating about a horizontal axis X with respect to a manipulation table 21 fixed to the floor; treatment manipulation portions 23 that are provided at right and left sides of the handle 22, that the operator O grasps and manipulates distal end portions thereof with his/her hands, and that have articulated structures corresponding to the treatment tools 9; and a foot switch 24 that is disposed on the floor.

As shown in Fig. 1, an assistant (not shown) lays the patient P on an operating table 30 that is disposed close to the master device 2 and performs proper treatment, such as sterilization and anesthesia.

The operator O directs the assistant to introduce the insertion portion 3 of the endoscope 4 into the large intestine from the anus of the patient P. The operator O manipulates the master device 2 to properly bend the bending portions of the insertion portion 3, thus moving the distal end of the endoscope 4.

The control portion 6 associates a rotation angle of the handle 22 of the master device 2 with a rotation angle of the distal-end surface 3a of the insertion portion 3 about a longitudinal axis Y and also associates manipulations of the treatment manipulation portions 23 of the master device 2 with movements of the treatment tools 9. Here, associating manipulation with movement means that manipulation performed at the master device 2 causes the corresponding portion of the endoscope 4 to move.

Specifically, the control portion 6 generates a rotation command signal for rotating the insertion portion 3 about the longitudinal axis Y according to the rotation angle of the handle 22 and outputs the rotation command signal to the driving portion 5. Furthermore, the control portion 6 generates rotation command signals for rotating corresponding joints of the treatment tools 9, according to the rotation angles of respective joints of the treatment manipulation portions 23 rotated by manipulating the treatment manipulation portions 23, and outputs the rotation command signals to the driving portion 5.

Furthermore, the control portion 6 includes an image processing portion (not shown) that corrects an image acquired via the objective lens 8 and outputs the corrected image to the monitor 7.

When the distal-end surface 3a of the insertion portion 3 is rotated by rotating the handle 22 about the horizontal axis X, the image processing portion rotates the image by the corresponding rotation angle, thereby generating a corrected image.

The function of the thus-configured master-slave system 1 of this embodiment will be described below.

With the insertion portion 3 of the endoscope 4 having been inserted into a body cavity, in order to observe the inside of the body cavity and perform treatment using the endoscope 4, the operator O manipulates, while observing the state of the body cavity captured by the endoscope 4 on the monitor 7, the handle 22 and the treatment manipulation portions 23 of the master device 2, thus moving the insertion portion 3 and the treatment tools 9 of the endoscope 4, which serves as the slave device.

From a state in which the handle 22 and the treatment tools 9 are located at standard positions as shown in Figs. 4(a) to 4(c), when the handle 22 is rotated as shown in Fig. 5, the bending portions of the insertion portion 3 of the endoscope 4 are moved to rotate the distal-end surface 3a about the longitudinal axis Y by the angle corresponding to (for example, the angle equal to) the rotation angle of the handle 22. When the distal-end surface 3a is rotated, the objective lens 8 and the treatment tools 9, which are provided on the distal-end surface 3a, are simultaneously rotated in the same direction.

When the objective lens 8 is rotated, the viewing field is rotated, and thus a subject A in an acquired image is rotated by the same rotation angle in the opposite direction from the rotation direction of the viewing field. Because the relative positions of the objective lens 8 and the treatment tools 9, which are disposed on the same distal-end surface 3a, are not changed, the positions of the treatment tools 9, which are partially in the acquired image, are not changed.

To deal with this, in this embodiment, the image processing portion, which is provided in the control portion 6, generates a corrected image obtained by rotating an image acquired via the objective lens 8 by the rotation angle of the insertion portion 3. The rotation direction of the image is the same as the rotation direction of the viewing field.

For example, starting from a position serving as a reference (standard position) shown in Figs. 4(a) to 4(c), when the handle 22 is rotated by an angle B in the counterclockwise direction, as shown in Fig. 5(a) and Fig. 6 (Step S1), the distal-end surface 3a of the insertion portion 3 is rotated about the longitudinal axis Y by the angle B in the counterclockwise direction, as shown in Fig. 5(b) (Step S2), and the viewing field is rotated by the angle B in the counterclockwise direction. As a result, the subject A in the acquired image is rotated by the angle B in the clockwise direction.

At this time, the control portion 6 calculates the angle B (Step S3), and the image processing portion generates a corrected image obtained by rotating the image by the angle B in the counterclockwise direction (Step S4) and displays the corrected image on the monitor 7 (Step S5). As a result, in the corrected image displayed on the monitor 7, as shown in Fig. 5(c), the subject A is not rotated, and the treatment tools 9 are rotated by the angle B in the counterclockwise direction.

Specifically, in this embodiment, when the operator O rotates the handle 22 by the angle B in the counterclockwise direction, the treatment tools 9 are also rotated by the angle B in the counterclockwise direction on the monitor 7; therefore, the operator O, who performs treatment while viewing the monitor 7, can perform intuitive manipulation. Furthermore, because the position of the handle 22 is matched with the positions of the treatment tools 9 on the monitor 7, even when the operator O leaves that place after performing manipulation and then performs manipulation again after a while, the operator O can easily recognize the association between the handle 22 and the treatment tools 9.

In the case of a conventional master-slave system in which image correction is not performed, as shown in Figs. 7(a) to 7(c), the treatment tools 9 in an image displayed on the monitor 7 are always fixed at the standard position, as shown in Fig. 7(c), irrespective of the rotation angle of the handle 22; therefore, as the rotation angle of the handle 22 is increased, the positional relationship between the handle 22 and the treatment tools 9 shows mismatching, thus causing a disadvantage in that it is difficult to recognize the association when manipulation is performed after a while.

Note that, in this embodiment, although a description has been given of a case in which the treatment manipulation portions 23 can be manipulated when the handle 22 is manipulated, the manipulation may be exclusively allowed in order to prevent a situation in which the treatment tools 9 are moved by mistake when the handle 22 is manipulated. In this case, as shown in Fig. 8, for example, a mode is selected using the foot switch 24 (Step S6): if the mode is switched to a treatment-tool manipulating mode, manipulation of the handle 22 is fixed (Step S7), and the treatment manipulation portions 23 are manipulated (Step S8), thereby moving the treatment tools 9 (Step S9). Furthermore, if the mode is switched to an endoscope manipulating mode, the treatment manipulation portions 23 are fixed (Step S10), the handle 22 is manipulated to move the insertion portion 3, and a corrected image is generated based on the rotation angle of the handle 22 and is displayed on the monitor 7 (Steps S1 to S5). Furthermore, it is possible to switch between the modes (Steps S11 and S12).

Furthermore, in this embodiment, although the image is corrected so as to be rotated whenever the insertion portion 3 is rotated, instead of this, switching may be allowed between a mode for rotating the image and a mode for not rotating the image according to a preference of the operator O.

Furthermore, as shown in Figs. 9(a) and 9(b), the master device 2 and the endoscope 4, which serves as a slave device, do not need to have similar shapes. In this case, an axis X is set at a center position between two grip portions 2a and 2b provided in the master device 2, and the rotation angle about this axis can be used as the rotation angle of the handle 22.

Furthermore, when a corrected image is generated, the image can be rotated about the center position of the image or about the center position of the subject A in the image. When the image is rotated about the center position of the subject A, the subject A can be displayed as if the subject A remains stationary in the corrected image.

Furthermore, in this embodiment, although a description has been given of an example case in which the rotation angle of the handle is equal to the rotation angle of the distal end of the insertion portion, these rotation angles may be different from each other as long as they are associated with each other. In that case, the image on the monitor is rotated by the rotation angle of the insertion portion, thereby making it possible to display the subject so as to be stationary. On the other hand, the image on the monitor is rotated by the rotation angle of the handle, thereby making it possible to match the position of the handle with the positions of the treatment tools on the monitor.

Next, a master-slave system according to a second embodiment of the present invention will be described with reference to the drawings. In the description of this embodiment, identical reference signs are assigned to portions having the same configurations as those of the master-slave device of the above-described first embodiment, and a description thereof will be omitted.

As shown in Fig. 10, in the master-slave system of this embodiment, as in the first embodiment, the control portion 6 rotates the image according to the rotation angle of the handle 22 and displays it on the monitor 7 (Steps S1 to S5), and, when the handle 22 is rotated from the standard position up to a set angle, for example, ±90° (Step S13), the following processing is performed.

Specifically, first, the control portion 6 displays a question about the need to rotate the image on the monitor 7 (Step S14).

In response to this, when the operator O performs input allowing the rotation, for example, using the foot switch 24, the control portion 6 gives an instruction to rotate the corrected image by a preset correction angle (for example, 180°). Accordingly, in the image on the monitor 7, the subject A is turned upside down, and the positions of the treatment tools 9 in the image are moved, for example, from the 90° position with respect to the standard position shown in Fig. 11(a) to the -90° position shown in Fig. 11(b) (Step S15) .

Next, the control portion 6 releases the association between the master device 2 and the endoscope 4 (Step S16). Accordingly, the connection is cut such that the endoscope 4 is not moved even when the operator O manipulates the master device 2. Then, in the above-described example, the insertion portion 3 of the endoscope 4 is maintained at the 90°-rotated position with respect to the standard position, and the image on the monitor 7 is also fixed so as not to be rotated from the -90° position.

Furthermore, when the handle 22 is manipulated (Step S17), the control portion 6 monitors the rotation angle of the handle 22 and determines whether or not the rotation angle of the handle 22 is matched with the rotation angle of the treatment tools 9 in the image on the monitor 7 (Step S18). In the above-described example, it is determined whether or not the rotation angle of the handle 22 has been moved from 90° to -90°.

Then, if the rotation angle of the handle 22 is matched with the rotation angle of the treatment tools 9 in the image on the monitor 7, the control portion 6 resumes the association between the master device 2 and the endoscope 4 (Step S19).

Accordingly, when the operator O manipulates the master device 2, the endoscope 4 is moved in response.

In this case, in the above-described example, because a state in which the insertion portion 3 of the endoscope 4 is rotated from the standard position by 90° is associated with a state in which the handle 22 is rotated from the standard position by -90°, when the handle 22 is rotated in the positive direction, the insertion portion 3 of the endoscope 4 can be rotated from the standard position to a position beyond the set angle of 90°.

Then, in this case, because the rotation angle of the handle 22 of the master device 2 is matched with the rotation angle of the treatment tools 9 on the monitor 7, the operator O, who performs treatment while viewing the monitor 7, has the advantage of being able to perform intuitive manipulation.

Furthermore, according to this embodiment, there is the advantage that the endoscope 4 can be moved over a wide angular range beyond the limits of the set angle, while preventing a cramped posture of the operator O, by keeping the rotation angle of the handle 22 within the limits of the set angle.

Note that, in this embodiment, although an example case in which the set angle, at which processing performed by the control portion 6 is switched, is set to ±90° is shown, the set angle is not limited thereto. Furthermore, although an example case in which the correction angle, by which an image is rotated when the rotation angle of the handle 22 exceeds the set angle, is set to 180° is shown, the correction angle is not limited thereto.

For example, when ±60° is set as the set angle, an angle from which the absolute value of the rotation angle of the handle 22 can be subtracted, which is equal to or lower than 120°, for example, an angle of 110° or -110°, is preferably adopted as the correction angle. Specifically, when the rotation angle of the handle 22 is rotated from the standard position by +60°, an angle of -110° is adopted as the correction angle, thereby rotating the corrected image on the monitor 7 from the standard position up to a rotation angle of -50°.

Therefore, after the association between the handle 22 and the insertion portion 3 is released, the association is resumed when the handle 22 is rotated up to a rotation angle of -50°, thereby making it possible to resume manipulation with the position of the handle 22 being matched with the positions of the treatment tools 9 on the monitor 7 and to rotate the insertion portion 3 over a wide range beyond the limits of the set angle.

Furthermore, in this embodiment, although the association is resumed when the handle 22 is rotated exactly by the correction angle, instead of this, the association may be resumed when the difference from the correction angle becomes equal to or lower than a predetermined threshold.

Next, a master-slave system according to a comparative example will be described below with reference to the drawings.

In the description of the master-slave system of this comparative example, identical reference signs are assigned to portions having the same configurations as those of the master-slave system of the above-described second embodiment, and a description thereof will be omitted.

As shown in Fig. 12, in the master-slave system of this comparative example, as in the second embodiment, the control portion 6 rotates the image according to the rotation angle of the handle 22 and displays it on the monitor 7 (Steps S1 to S5), and, when the handle 22 is rotated up to a arbitrary angle, and when the operator O performs input by manipulating the foot switch 24 (Step S20), the manipulation of the treatment manipulation portions 23 is disabled in response to the input (Step S21), and the association between the master device 2 and the endoscope 4 is released (Step S22).

Next, after the handle 22 is moved in a reverse direction by the arbitrary angle (movement angle) (Step S23), when the operator O performs input by manipulating the foot switch 24 again (Step S24), the control portion 6 moves the corrected image on the monitor 7 by the above-described movement angle (Step S25), resumes the association (Step S26), and enables the manipulation of the treatment manipulation portions 23 (Step S27).

For example, when the operator O manipulates the foot switch 24 in a state in which the handle 22 is rotated from the standard position by 45°, because the association between the master device 2 and the endoscope 4 is released, the insertion portion 3 of the endoscope 4 is maintained at the rotation angle of 45°, and the handle 22 can be freely rotated. The control portion 6 monitors the movement angle of the handle 22 from a point in time at which the foot switch 24 is manipulated, and, when the operator O rotates the handle 22, for example, by -60° and manipulates the foot switch 24 again, the corrected image on the monitor 7 is rotated by the movement angle (-60°).

Accordingly, because the position of the handle 22 is matched with the positions of the treatment tools 9 on the monitor 7, the control portion 6 resumes the association between the master device 2 and the endoscope 4, thereby making it possible to continue intuitive manipulation and to move the endoscope 4 over a wider angular range while preventing a cramped posture of the operator O.

Next, a master-slave system according to a third embodiment of the present invention will be described below with reference to the drawings.

In the description of this embodiment, identical reference signs are assigned to portions having the same configurations as those of the master-slave system 1 of the above-described first embodiment, and a description thereof will be omitted.

As shown in Fig. 13, this embodiment differs from the master-slave systems 1 of the first and second embodiments in that the monitor 7 is fixed to the handle 22, and correction processing for rotating an image is not performed.

Specifically, in this embodiment, when the handle 22 is rotated from the standard position shown in Fig. 14, the treatment tools 9 displayed on the monitor 7 are not moved with respect to the monitor 7, as shown in Fig. 15, but the monitor 7 is moved together with the handle 22; therefore, the angle of the treatment tools 9 displayed on the monitor 7 is matched with the angle of the handle 22 in practice.

Therefore, in the master-slave system of this embodiment, the operator O can perform intuitive manipulation while viewing the monitor 7.

Note that, even when the monitor 7 is fixed to the handle 22, if the rotation angle of the handle 22 is increased, the posture of the operator O becomes cramped; therefore, this can be avoided by using the following method.

When the handle 22 is rotated from the standard position at the rotation angle of 0° up to a set angle, for example, ±90°, the control portion 6 asks the operator O whether or not he/she desires to return the position of the handle 22. If the operator O performs input indicating that he/she desires to return the position of the handle 22, for example, using the foot switch 24, the control portion 6 releases the association between the master device 2 and the endoscope 4 and resumes the association between the master device 2 and the endoscope 4 when the operator O returns the handle 22 to the vicinity of the standard position.

Furthermore, the following method can also be adopted as a method of allowing intuitive manipulation while preventing a cramped posture caused by manipulating the handle 22.

Specifically, first, from the state of the standard position shown in Figs. 16(a) and 16(b), the operator O rotates the handle 22 by a arbitrary movement angle, as shown in Figs. 17(a) and 17(b), without generating a corrected image. At this time, the positional relationship between the handle 22 and the treatment tools 9 displayed on the monitor 7 shows mismatching.

In this state, when the operator O steps on the foot switch 24, the association between the master device 2 and the endoscope 4 is released, and, when the handle 22 is returned by the above-described movement angle, as shown in Figs. 18(a) and 18(b), the association is resumed. Accordingly, the positional relationship between the handle 22 and the treatment tools 9 displayed on the monitor 7 shows matching. Therefore, by repeating this, while manipulating the handle 22 within a relatively narrow angular range, the treatment tools can be rotated over a wider angular range than that angular range.

### {Reference Signs List}

- A: subject
- X: horizontal axis (axis)
- Y: longitudinal axis (axis)
- 1: master-slave system
- 3a: distal-end surface (surface)
- 4: endoscope (slave device)
- 6: control portion
- 7: monitor
- 8: objective lens (observation optical system)
- 9: treatment tool (treatment portion)
- 22: handle
- 23: treatment manipulation portion
- 24: foot switch (input portion)
- S2: movement step
- S4: image generating step
- S5: display step
- S16: release step
- S19: resumption step

## Claims

1. A master-slave system (1) comprising:
an endoscope (4) that includes an observation optical system (8) that photographs a subject (A) and a treatment portion (9) that protrudes from a surface on which the observation optical system (8) is provided and that is at least partially photographed by the observation optical system (8) together with the subject (A);
a master device (2) that includes a handle (22) that is supported so as to be capable of rotating about a predetermined axis and a treatment manipulation portion (23) that is provided on the handle (22);
a control portion (6) that associates rotation of the handle (22) of the master device (2) with rotation of the endoscope (4) about an axis intersecting the surface and that associates manipulation of the treatment manipulation portion (23) with movement of the treatment portion (9);
an image processing portion that generates a corrected image obtained by rotating an image acquired by the observation optical system (8) by a rotation angle of the endoscope (4) about the axis intersecting the surface; and
a monitor (7) that displays the corrected image generated by the image processing portion,
wherein, when the handle (22) is rotated up to a set angle, the control portion (6) is configured to switch from the corrected image on the monitor (7) to a re-corrected image obtained by additionally rotating the corrected image by a predetermined correction angle from which the absolute value of the rotation angle of the handle (22) can be subtracted, release the association between rotation of the handle (22) of the master device (2) and rotation of the endoscope (4) about the axis intersecting the surface, and resume the association when the handle (22) is moved by an angle whose difference from the correction angle is equal to or lower than a predetermined threshold.

2. A master-slave system (1) comprising:
an endoscope (4) that includes an observation optical system (8) that photographs a subject (A) and a treatment portion (9) that protrudes from a surface on which the observation optical system (8) is provided and that is at least partially photographed by the observation optical system (8) together with the subject (A);
a master device (2) that includes a handle (22) that is supported so as to be capable of rotating about a predetermined axis and a treatment manipulation portion (23) that is provided on the handle (22);
a control portion (6) that associates rotation of the handle (22) of the master device (2) with rotation of the endoscope (4) about an axis intersecting the surface and that associates manipulation of the treatment manipulation portion (23) with movement of the treatment portion (9);
an image processing portion that generates a corrected image obtained by rotating an image acquired by the observation optical system (8) by a rotation angle of the handle (22); and
a monitor (7) that displays the corrected image generated by the image processing portion;
wherein, when the handle (22) is rotated up to a set angle, the control portion (6) is configured to switch from the corrected image on the monitor (7) to a re-corrected image obtained by additionally rotating the corrected image by a predetermined correction angle from which the absolute value of the rotation angle of the handle (22) can be subtracted, release the association between rotation of the handle (22) of the master device (2) and rotation of the endoscope (4) about the axis intersecting the surface, and resume the association when the handle (22) is moved by an angle whose difference from the correction angle is equal to or lower than a predetermined threshold.

3. A master-slave system (1) comprising:
an endoscope (4) that includes an observation optical system (8) that photographs a subject (A) and a treatment portion (9) that protrudes from a surface on which the observation optical system (8) is provided and that is at least partially photographed by the observation optical system (8) together with the subject (A);
a master device (2) that includes a handle (22) that is supported so as to be capable of rotating about a predetermined axis and a treatment manipulation portion (23) that is provided on the handle (22);
a control portion (6) that associates rotation of the handle (22) of the master device (2) with rotation of the endoscope (4) about an axis intersecting the surface and that associates manipulation of the treatment manipulation portion (23) with movement of the treatment portion (9);
an image processing portion that generates a corrected image obtained by rotating an image acquired by the observation optical system (8) by a rotation angle of the endoscope (4) about the axis intersecting the surface; and
a monitor (7) that displays the corrected image generated by the image processing portion;
wherein the control portion (6) is configured to release the association between rotation of the handle (22) and rotation of the endoscope (4) about the axis intersecting the surface, with the handle (22) having been rotated, rotate, when the handle (22) is then moved by an arbitrary movement angle, the corrected image displayed on the monitor (7) by the movement angle, and resume the association.

4. A master-slave system (1) comprising:
an endoscope (4) that includes an observation optical system (8) that photographs a subject (A) and a treatment portion (9) that protrudes from a surface on which the observation optical system (8) is provided and that is at least partially photographed by the observation optical system (8) together with the subject (A);
a master device (2) that includes a handle (22) that is supported so as to be capable of rotating about a predetermined axis and a treatment manipulation portion (23) that is provided on the handle (22);
a control portion (6) that associates rotation of the handle (22) of the master device (2) with rotation of the endoscope (4) about an axis intersecting the surface and that associates manipulation of the treatment manipulation portion (23) with movement of the treatment portion (9);
an image processing portion that generates a corrected image obtained by rotating an image acquired by the observation optical system (8) by a rotation angle of the handle (22); and
a monitor (7) that displays the corrected image generated by the image processing portion,
wherein the control portion (6) is configured to release the association between rotation of the handle (22) and rotation of the endoscope (4) about the axis intersecting the surface, with the handle (22) having been rotated, rotate, when the handle (22) is then moved by an arbitrary movement angle, the corrected image displayed on the monitor (7) by the movement angle, and resume the association.

5. The master-slave system (1) according to one of claims 1 to 4, wherein the image processing portion generates the corrected image obtained by rotating an image acquired by the observation optical system (8) about the center of the image.

6. The master-slave system (1) according to one of claims 1 to 4, wherein the image processing portion generates the corrected image obtained by rotating an image acquired by the observation optical system (8) about an axis that is fixed relative to the subject in the image.

7. A master-slave system (1) comprising:
a endoscope (4) that includes an observation optical system (8) that photographs a subject (A) and a treatment portion (9) that protrudes from a surface on which the observation optical system (8) is provided and that is at least partially photographed by the observation optical system (8) together with the subject (A);
a master device (2) that includes a handle (22) that is supported so as to be capable of rotating about a predetermined axis and a treatment manipulation portion (23) that is provided on the handle (22); and
a control portion (6) that associates rotation of the handle (22) of the master device (2) with rotation of the endoscope (4) about an axis intersecting the surface and that associates manipulation of the treatment manipulation portion (23) with movement of the treatment portion (9);
**characterized in that** the master-slave system (1) further includes a monitor (7) that is fixed to the handle (22) and that displays an image acquired by the observation optical system (8).

8. The master-slave system (1) according to claim 7, wherein the control portion (6) releases the association between rotation of the handle (22) of the master device (2) and rotation of the endoscope (4) about the axis intersecting the surface when the handle (22) is rotated from a standard position, at which the handle (22) has not been rotated, up to a set angle and resumes the association when the handle (22) is returned to the standard position.

## Patentansprüche

1. Master-Slave-System (1), umfassend:
ein Endoskop (4), das ein optisches Beobachtungssystem (8) umfasst, das ein Subjekt (A) fotografiert, und einen Behandlungsabschnitt (9), der von einer Oberfläche, auf der das optische Beobachtungssystem (8) bereitgestellt ist, vorsteht und der mindestens teilweise von dem optischen Beobachtungssystem (8) zusammen mit dem Subjekt (A) fotografiert wird;
eine Master-Vorrichtung (2), die einen Griff (22) umfasst, der derart gelagert ist, dass er um eine vorbestimmte Achse drehen kann, und einen Behandlungsmanipulationsabschnitt (23), der an dem Griff (22) bereitgestellt ist;
einen Steuerungsabschnitt (6), der die Drehung des Griffs (22) der Master-Vorrichtung (2) mit der Drehung des Endoskops (4) um eine die Oberfläche schneidende Achse verknüpft, und der die Manipulation des Behandlungsmanipulationsabschnitts (23) mit der Bewegung des Behandlungsabschnitts (9) verknüpft;
einen Bildverarbeitungsabschnitt, der ein korrigiertes Bild erzeugt, das durch Drehen eines von dem optischen Beobachtungssystem (8) erfassten Bildes um einen Drehwinkel des Endoskops (4) um die die Oberfläche schneidende Achse erhalten wird; und
einen Bildschirm (7), der das von dem Bildverarbeitungsabschnitt erzeugte korrigierte Bild anzeigt,
wobei, wenn der Griff (22) bis zu einem eingestellten Winkel gedreht wird, der Steuerungsabschnitt (6) dazu ausgelegt ist, von dem korrigierten Bild auf dem Bildschirm (7) auf ein neu korrigiertes Bild zu schalten, das erhalten wird durch zusätzliches Drehen des korrigierten Bildes um einen vorbestimmten Korrekturwinkel, von dem der absolute Wert des Drehwinkels des Griffs (22) subtrahiert werden kann, Lösen der Verknüpfung zwischen der Drehung des Griffs (22) der Master-Vorrichtung (2) und der Drehung des Endoskops (4) um die die Oberfläche schneidende Achse und Wiederaufnehmen der Verknüpfung, wenn der Griff (22) um einen Winkel bewegt wird, dessen Differenz von dem Korrekturwinkel gleich oder kleiner ist als ein vorbestimmter Schwellwert.

2. Master-Slave-System (1), umfassend:
ein Endoskop (4), das ein optisches Beobachtungssystem (8) umfasst, das ein Subjekt (A) fotografiert, und einen Behandlungsabschnitt (9), der von einer Oberfläche, auf der das optische Beobachtungssystem (8) bereitgestellt ist, vorsteht und der mindestens teilweise von dem optischen Beobachtungssystem (8) zusammen mit dem Subjekt (A) fotografiert wird;
eine Master-Vorrichtung (2), die einen Griff (22) umfasst, der derart gelagert ist, dass er um eine vorbestimmte Achse drehen kann, und einen Behandlungsmanipulationsabschnitt (23), der an dem Griff (22) bereitgestellt ist;
einen Steuerungsabschnitt (6), der die Drehung des Griffs (22) der Master-Vorrichtung (2) mit der Drehung des Endoskops (4) um eine die Oberfläche schneidende Achse verknüpft und der die Manipulation des Behandlungsmanipulationsabschnitts (23) mit der Bewegung des Behandlungsabschnitts (9) verknüpft;
einen Bildverarbeitungsabschnitt, der ein korrigiertes Bild erzeugt, das durch Drehen eines von dem optischen Beobachtungssystem (8) erfassten Bildes um einen Drehwinkel des Griffs (22) erhalten wurde; und
einen Bildschirm (7), der das von dem Bildverarbeitungsabschnitt erzeugte korrigierte Bild anzeigt;
wobei, wenn der Griff (22) bis zu einem eingestellten Winkel gedreht wird, der Steuerungsabschnitt (6) dazu ausgelegt ist, von dem korrigierten Bild auf dem Bildschirm (7) auf ein neu korrigiertes Bild zu schalten, das erhalten wird durch zusätzliches Drehen des korrigierten Bildes um einen vorbestimmten Korrekturwinkel, von dem der absolute Wert des Drehwinkels des Griffs (22) subtrahiert werden kann, Lösen der Verknüpfung zwischen der Drehung des Griffs (22) der Master-Vorrichtung (2) und der Drehung des Endoskops (4) um die die Oberfläche schneidende Achse und Wiederaufnehmen der Verknüpfung, wenn der Griff (22) um einen Winkel bewegt wird, dessen Differenz von dem Korrekturwinkel gleich oder kleiner ist als ein vorbestimmter Schwellwert.

3. Master-Slave-System (1), umfassend:
ein Endoskop (4), das ein optisches Beobachtungssystem (8) umfasst, das ein Subjekt (A) fotografiert, und einen Behandlungsabschnitt (9), der von einer Oberfläche, auf der das optische Beobachtungssystem (8) bereitgestellt ist, vorsteht und der mindestens teilweise von dem optischen Beobachtungssystem (8) zusammen mit dem Subjekt (A) fotografiert wird;
eine Master-Vorrichtung (2), die einen Griff (22) umfasst, der derart gelagert ist, dass er um eine vorbestimmte Achse drehen kann, und einen Behandlungsmanipulationsabschnitt (23), der an dem Griff (22) bereitgestellt ist;
einen Steuerungsabschnitt (6), der die Drehung des Griffs (22) der Master-Vorrichtung (2) mit der Drehung des Endoskops (4) um eine die Oberfläche schneidende Achse verknüpft und der die Manipulation des Behandlungsmanipulationsabschnitts (23) mit der Bewegung des Behandlungsabschnitts (9) verknüpft;
einen Bildverarbeitungsabschnitt, der ein korrigiertes Bild erzeugt, das durch Drehen eines von dem optischen Beobachtungssystem (8) erfassten Bildes um einen Drehwinkel des Endoskops (4) um die die Oberfläche schneidende Achse erhalten wurde; und
einen Bildschirm (7), der das von dem Bildverarbeitungsabschnitt erzeugte korrigierte Bild anzeigt;
wobei der Steuerungsabschnitt (6) dazu ausgelegt ist, die Verknüpfung zwischen der Drehung des Griffs (22) und der Drehung des Endoskops (4) um die die Oberfläche schneidende Achse zu lösen, das auf dem Bildschirm (7) angezeigte korrigierte Bild bei gedrehtem Griff (22), wenn der Griff (22) dann um einen beliebigen Bewegungswinkel bewegt wird, um den Bewegungswinkel zu drehen und die Verknüpfung wieder aufzunehmen.

4. Master-Slave-System (1), umfassend:
ein Endoskop (4), das ein optisches Beobachtungssystem (8) umfasst, das ein Subjekt (A) fotografiert, und einen Behandlungsabschnitt (9), der von einer Oberfläche, auf der das optische Beobachtungssystem (8) bereitgestellt ist, vorsteht und der mindestens teilweise von dem optischen Beobachtungssystem (8) zusammen mit dem Subjekt (A) fotografiert wird;
eine Master-Vorrichtung (2), die einen Griff (22) umfasst, der derart gelagert ist, dass er um eine vorbestimmte Achse drehen kann, und einen Behandlungsmanipulationsabschnitt (23), der an dem Griff (22) bereitgestellt ist;
einen Steuerungsabschnitt (6), der die Drehung des Griffs (22) der Master-Vorrichtung (2) mit der Drehung des Endoskops (4) um eine die Oberfläche schneidende Achse verknüpft und der die Manipulation des Behandlungsmanipulationsabschnitts (23) mit der Bewegung des Behandlungsabschnitts (9) verknüpft;
einen Bildverarbeitungsabschnitt, der ein korrigiertes Bild erzeugt, das durch Drehen eines von dem optischen Beobachtungssystem (8) erfassten Bildes um einen Drehwinkel des Griffs (22) erhalten wurde; und
einen Bildschirm (7), der das von dem Bildverarbeitungsabschnitt erzeugte korrigierte Bild anzeigt,
wobei der Steuerungsabschnitt (6) dazu ausgelegt ist, die Verknüpfung zwischen der Drehung des Griffs (22) und der Drehung des Endoskops (4) um die die Oberfläche schneidende Achse zu lösen, das auf dem Bildschirm (7) angezeigte korrigierte Bild bei gedrehtem Griff (22), wenn der Griff (22) dann um einen beliebigen Bewegungswinkel bewegt wird, um den Bewegungswinkel zu drehen und die Verknüpfung wieder aufzunehmen.

5. Master-Slave-System (1) nach einem der Ansprüche 1 bis 4, wobei der Bildverarbeitungsabschnitt das korrigierte Bild erzeugt, das durch Drehen eines von dem optischen Beobachtungssystem (8) erfassten Bildes um das Zentrum des Bildes erhalten wurde.

6. Master-Slave-System (1) nach einem der Ansprüche 1 bis 4, wobei der Bildverarbeitungsabschnitt das korrigierte Bild erzeugt, das durch Drehen eines von dem optischen Beobachtungssystem (8) erfassten Bildes um eine Achse, die bezogen auf Subjekt in dem Bild befestigt ist, erhalten wurde.

7. Master-Slave-System (1), umfassend:
ein Endoskop (4), das ein optisches Beobachtungssystem (8) umfasst, das ein Subjekt (A) fotografiert, und einen Behandlungsabschnitt (9), der von einer Oberfläche, auf der das optische Beobachtungssystem (8) bereitgestellt ist, vorsteht und der mindestens teilweise von dem optischen Beobachtungssystem (8) zusammen mit dem Subjekt (A) fotografiert wird;
eine Master-Vorrichtung (2), die einen Griff (22) umfasst, der derart gelagert ist, dass er um eine vorbestimmte Achse drehen kann, und einen Behandlungsmanipulationsabschnitt (23), der an dem Griff (22) bereitgestellt ist; und
einen Steuerungsabschnitt (6), der die Drehung des Griffs (22) der Master-Vorrichtung (2) mit der Drehung des Endoskops (4) um eine die Oberfläche schneidende Achse verknüpft und der die Manipulation des Behandlungsmanipulationsabschnitts (23) mit der Bewegung des Behandlungsabschnitts (9) verknüpft;
**dadurch gekennzeichnet, dass** das Master-Slave-System (1) ferner einen Bildschirm (7) umfasst, der an dem Griff (22) befestigt ist und der ein von dem optischen Beobachtungssystem (8) erfasstes Bild anzeigt.

8. Master-Slave-System (1) nach Anspruch 7, wobei der Steuerungsabschnitt (6) die Verknüpfung zwischen der Drehung des Griffs (22) der Master-Vorrichtung (2) und der Drehung des Endoskops (4) um die die Oberfläche schneidende Achse löst, wenn der Griff (22) aus einer Standardposition, in der der Griff (22) nicht gedreht wurde, bis zu einem eingestellten Winkel gedreht wird, und die Verknüpfung wieder aufnimmt, wenn der Griff (22) in die Standardposition zurückgedreht wird.

## Revendications

1. Système maître-esclave (1) comprenant :
un endoscope (4) qui comprend un système optique d'observation (8) qui photographie un sujet (A) et une partie de traitement (9) qui fait saillie à partir d'une surface sur laquelle se trouve le système optique d'observation (8) et qui est au moins partiellement photographiée par le système optique d'observation (8) conjointement avec le sujet (A) ;
un dispositif maître (2) qui comprend une poignée (22) qui est supportée de façon à être capable de tourner autour d'un axe prédéterminé et une partie de manipulation de traitement (23) qui se trouve sur la poignée (22) ;
une partie de commande (6) qui associe une rotation de la poignée (22) du dispositif maître (2) à une rotation de l'endoscope (4) autour d'un axe coupant la surface, et qui associe une manipulation de la partie de manipulation de traitement (23) à un mouvement de la partie de traitement (9) ;
une partie de traitement d'image qui génère une image corrigée obtenue par rotation d'une image acquise par le système optique d'observation (8) d'un angle de rotation de l'endoscope (4) autour de l'axe coupant la surface ; et
un moniteur (7) qui affiche l'image corrigée générée par la partie de traitement d'image,
dans lequel, lorsque la poignée (22) est tournée vers le haut à un angle défini, la partie de commande (6) est configurée pour commuter de l'image corrigée sur le moniteur (7) à une image re-corrigée obtenue par rotation supplémentaire de l'image corrigée d'un angle de correction prédéterminé auquel la valeur absolue de l'angle de rotation de la poignée (22) peut être soustraite, suspendre l'association entre une rotation de la poignée (22) du dispositif maître (2) et une rotation de l'endoscope (4) autour de l'axe coupant la surface, et reprendre l'association lorsque la poignée (22) est déplacée d'un angle dont une différence par rapport à l'angle de correction est inférieure ou égale à un seuil prédéterminé.

2. Système maître-esclave (1) comprenant :
un endoscope (4) qui comprend un système optique d'observation (8) qui photographie un sujet (A) et une partie de traitement (9) qui fait saillie à partir d'une surface sur laquelle se trouve le système optique d'observation (8) et qui est au moins partiellement photographiée par le système optique d'observation (8) conjointement avec le sujet (A) ;
un dispositif maître (2) qui comprend une poignée (22) qui est supportée de façon à être capable de tourner autour d'un axe prédéterminé et une partie de manipulation de traitement (23) qui se trouve sur la poignée (22) ;
une partie de commande (6) qui associe une rotation de la poignée (22) du dispositif maître (2) à une rotation de l'endoscope (4) autour d'un axe coupant la surface, et qui associe une manipulation de la partie de manipulation de traitement (23) à un mouvement de la partie de traitement (9) ;
une partie de traitement d'image qui génère une image corrigée obtenue par rotation d'une image acquise par le système optique d'observation (8) d'un angle de rotation de la poignée (22) ; et
un moniteur (7) qui affiche l'image corrigée générée par la partie de traitement d'image,
dans lequel, lorsque la poignée (22) est tournée vers le haut à un angle défini, la partie de commande (6) est configurée pour commuter de l'image corrigée sur le moniteur (7) à une image re-corrigée obtenue par rotation supplémentaire de l'image corrigée d'un angle de correction prédéterminé auquel la valeur absolue de l'angle de rotation de la poignée (22) peut être soustraite, suspendre l'association entre une rotation de la poignée (22) du dispositif maître (2) et une rotation de l'endoscope (4) autour de l'axe coupant la surface, et reprendre l'association lorsque la poignée (22) est déplacée d'un angle dont une différence par rapport à l'angle de correction est inférieure ou égale à un seuil prédéterminé.

3. Système maître-esclave (1) comprenant :
un endoscope (4) qui comprend un système optique d'observation (8) qui photographie un sujet (A) et une partie de traitement (9) qui fait saillie à partir d'une surface sur laquelle se trouve le système optique d'observation (8) et qui est au moins partiellement photographiée par le système optique d'observation (8) conjointement avec le sujet (A) ;
un dispositif maître (2) qui comprend une poignée (22) qui est supportée de façon à être capable de tourner autour d'un axe prédéterminé et une partie de manipulation de traitement (23) qui se trouve sur la poignée (22) ;
une partie de commande (6) qui associe une rotation de la poignée (22) du dispositif maître (2) à une rotation de l'endoscope (4) autour d'un axe coupant la surface, et qui associe une manipulation de la partie de manipulation de traitement (23) à un mouvement de la partie de traitement (9) ;
une partie de traitement d'image qui génère une image corrigée obtenue par rotation d'une image acquise par le système optique d'observation (8) d'un angle de rotation de l'endoscope (4) autour de l'axe coupant la surface ; et
un moniteur (7) qui affiche l'image corrigée générée par la partie de traitement d'image,
dans lequel la partie de commande (6) est configurée pour suspendre l'association entre une rotation de la poignée (22) et une rotation de l'endoscope (4) autour de l'axe coupant la surface, la poignée (22) ayant été tournée, tourner, lorsque la poignée (22) est ensuite déplacée d'un angle de mouvement arbitraire, l'image corrigée affichée sur le moniteur (7) de l'angle de mouvement, et reprendre l'association.

4. Système maître-esclave (1) comprenant :
un endoscope (4) qui comprend un système optique d'observation (8) qui photographie un sujet (A) et une partie de traitement (9) qui fait saillie à partir d'une surface sur laquelle se trouve le système optique d'observation (8) et qui est au moins partiellement photographiée par le système optique d'observation (8) conjointement avec le sujet (A) ;
un dispositif maître (2) qui comprend un poignée (22) qui est supportée de façon à être capable de tourner autour d'un axe prédéterminé et une partie de manipulation de traitement (23) qui se trouve sur la poignée (22) ;
une partie de commande (6) qui associe une rotation de la poignée (22) du dispositif maître (2) à une rotation de l'endoscope (4) autour d'un axe coupant la surface, et qui associe une manipulation de la partie de manipulation de traitement (23) à un mouvement de la partie de traitement (9) ;
une partie de traitement d'image qui génère une image corrigée obtenue par rotation d'une image acquise par le système optique d'observation (8) d'un angle de rotation de la poignée (22) ; et
un moniteur (7) qui affiche l'image corrigée générée par la partie de traitement d'image,
dans lequel la partie de commande (6) est configurée pour suspendre l'association entre une rotation de la poignée (22) et une rotation de l'endoscope (4) autour de l'axe coupant la surface, la poignée (22) ayant été tournée, tourner, lorsque la poignée (22) est ensuite déplacée d'un angle de mouvement arbitraire, l'image corrigée affichée sur le moniteur (7) de l'angle de mouvement, et reprendre l'association.

5. Système maître-esclave (1) selon l'une des revendications 1 à 4, dans lequel la partie de traitement d'image génère l'image corrigée obtenue par rotation d'une image acquise par le système optique d'observation (8) autour du centre de l'image.

6. Système maître-esclave (1) selon l'une des revendications 1 à 4, dans lequel la partie de traitement d'image génère l'image corrigée obtenue par rotation d'une image acquise par le système optique d'observation (8) autour d'un axe qui est fixe par rapport au sujet dans l'image.

7. Système maître-esclave (1) comprenant :
un endoscope (4) qui comprend un système optique d'observation (8) qui photographie un sujet (A) et une partie de traitement (9) qui fait saillie à partir d'une surface sur laquelle se trouve le système optique d'observation (8) et qui est au moins partiellement photographiée par le système optique d'observation (8) conjointement avec le sujet (A) ;
un dispositif maître (2) qui comprend une poignée (22) qui est supportée de façon à être capable de tourner autour d'un axe prédéterminé et une partie de manipulation de traitement (23) qui se trouve sur la poignée (22) ; et
une partie de commande (6) qui associe une rotation de la poignée (22) du dispositif maître (2) à une rotation de l'endoscope (4) autour d'un axe coupant la surface, et qui associe une manipulation de la partie de manipulation de traitement (23) à un mouvement de la partie de traitement (9) ;
**caractérisé par le fait que** le système maître-esclave (1) comprend en outre un moniteur (7) qui est fixé à la poignée (22) et qui affiche une image acquise par le système optique d'observation (8).

8. Système maître-esclave (1) selon la revendication 7, dans lequel la partie de commande (6) suspend l'association entre une rotation de la poignée (22) du dispositif maître (2) et une rotation de l'endoscope (4) autour de l'axe coupant la surface lorsque la poignée (22) est tournée à partir d'une position standard, dans laquelle la poignée (22) n'a pas été tournée, jusqu'à un angle défini et reprend l'association lorsque la poignée (22) est ramenée à la position standard.
